# EUROPEAN PATENT SPECIFICATION

(11) **EP 4 111 182 B1**
(45) Date of publication and mention of the grant of the patent: **19.03.2025**
(21) Application number: 21710561.8
(22) Date of filing: 17.02.2021
(51) Int. Cl.: G01N 23/046, G01N 3/60, G01N 33/38, G06T 7/00

(54) **METHOD FOR THE EXAMINATION OF THE FREEZE-THAW RESISTANCE OF CONCRETE STRUCTURES**
VERFAHREN ZUR UNTERSUCHUNG DER FROST-TAU-BESTÄNDIGKEIT VON BETONSTRUKTUREN
PROCÉDÉ D'EXAMEN DE LA RÉSISTANCE AU CYCLE GEL-DÉGEL DE STRUCTURES EN BÉTON

(30) Priority: 27.02.2020 HU 2000073
(43) Date of publication of application: 04.01.2023
(73) Proprietor: Budapesti Müszaki és Gazdaságtudományi Egyetem, 1111 Budapest (HU); SW Umwelttechnik Magyarország Kft, 2339 Majosháza (HU)
(72) Inventor: BALÁZS L., György, 1116 Budapest (HU); MAJOROSNÉ LUBLÓY, Éva Eszter, 1183 Budapest (HU); HLAVICKA, Viktor, 1133 Budapest (HU); NEHME, Salem G., 1225 Budapest (HU); KAPITÁNY, Kristóf, 1223 Budapest (HU); FÖLDES, Tamás, 5000 Szolnok (HU); LIZAKOVSZKY, Géza, 3530 Miskolc (HU); MOLNÁR, Tamás, 1191 Budapest (HU)
(74) Representative: Danubia Patent & Law Office LLC
(86) International application number: PCT/HU2021/050013
(87) International publication number: WO 2021/171047

(56) References cited:
- EP-A1- 0 010 777
- CHEN SHAOJIE ET AL: "Salt Freeze-Thaw Damage Characteristics of Concrete based on Computed Tomography", TEHNICKI VJESNIK, vol. 26, no. 6, 1 November 2019 (2019-11-01), HR, pages 1753 - 1763, XP055802874, ISSN: 1330-3651, DOI: 10.17559/TV-20190819080524
- DAUKSYS MINDAUGAS ET AL: "The Assessment of Prediction Methodology of Concrete Freezing and Thawing Resistance", MEDZIAGOTYRA - MATERIALS SCIENCE, vol. 18, no. 4, 17 December 2012 (2012-12-17), LT, XP055802998, ISSN: 1392-1320, DOI: 10.5755/j01.ms.18.4.3105
- NAGROCKIENE DZIGITA ET AL: "Predicting Frost Resistance of Concrete with Different Coarse Aggregate Concentration by Porosity Parameters", MEDZIAGOTYRA - MATERIALS SCIENCE, vol. 17, no. 2, 1 July 2011 (2011-07-01), LT, pages 203 - 207, XP055803007, ISSN: 1392-1320, Retrieved from the Internet <URL:http://matsc.ktu.lt/index.php/MatSc/article/download/493/419> DOI: 10.5755/j01.ms.17.2.493
- YUAN JIE ET AL: "Characterization of air voids and frost resistance of concrete based on industrial computerized tomographical technology", CONSTRUCTION AND BUILDING MATERIALS, vol. 168, 1 April 2018 (2018-04-01), Netherlands, pages 975 - 983, XP055803486, ISSN: 0950-0618, DOI: 10.1016/j.conbuildmat.2018.01.117

## Description

The invention relates to a method for the examination of the freeze-thaw resistance of concrete structures, in which one or more representative specimens of a predetermined size are prepared from the concrete structure, the inner structure of the specimen is examined by computer tomography, and the relative amount of a predetermined component is determined in the specimen by thresholding, following picture enhancement and picture correction carried out by a computer.

The freeze-thaw resistance of concrete structures has to be examined on specimens obtained from the structures by using the methods as defined in the European Standard CEN/TS 122390-9 which can be (A) freeze-thaw cycle exposure (B) lateral sealing and (C) capillary suction.

In case of the examinations according to conditions (A), the specimens are frozen under water or salty water after at least 28 days following the manufacturing of the concrete. The prescribed number of the freeze-thaw cycles is 56. The condition of satisfaction is: the loss of mass should be < 5 m% and the loss in strength should be < 20 %.

In examinations by lateral sealing, the prescribed number of the freeze-thaw cycles is 56. The condition of acceptance is the loss of mass, wherein its amount is defined according to the environmental conditions.

The capillary suction examinations are defined in the 2007 edition of the cited standard. In this case the examinations may only be started after at least 28 days have elapsed since the manufacturing date of the concrete. Based on the examinations, the following qualifications can be awarded: XF2, XF4; XF1, XF3. The codes relate to the direction of the effects of water. The condition of satisfactory results: the loss of mass should be under 1500 g/cm².

The results of such examinations will become available only after a substantial delay counted from the concrete structure's date of manufacturing, because the examinations cannot be started earlier than 28 days following the manufacture, and the respective freeze-thaw cycles are time consuming. Therefore, the result will not be available before 56 days, but in some cases, the delay is even longer. Furthermore, the freeze-thaw cycling and its evaluation are costly and require much work.

With regard to such drawbacks, several attempts have become known to the non-destructive examination of freeze-thaw resistance, but neither of them have obtained wide acceptance or use.

According to JP2003149233 the concrete specimen was immersed in a liquid having a high contrast property and this medium has penetrated into the micro scratches and failures in the interior of the concrete specimen. An X-Ray recording was made from the concrete specimen soaked in this way, whereby the micro scratches can be seen and the degree of the worsening was determined based on the amount of the micro-scratches which ware also displayed. The publication does not provide a clear figure on the freeze-thaw resistance of the examined specimen.

In the article: "Experimental Investigation of the Variation of Concrete Pores under the Action of Freeze-Thaw Cycles by Using X-Ray CT" by Jie JUAN et. al (Hindawi Publishing Corporation, Advances in Material Science and Engineering, Volume 2014, Article ID 5713657, 11 pages), also available at the web address http://dx.doi.org/10.1155/2014/571357, the results of expedited freeze-thaw examinations were reported which were carried out on cylindrical specimens with a diameter of 75 mm and a length of 150 mm. The examinations were carried out here by means of micro X-Ray CT devices having special micro focus, wherein the table could be moved around 5 axes. A special picture enhancement software was used in the measurements, and the specimens were examined in four concentric non-overlapping ring-like portions. Before the examinations, the specimens were soaked through several days in water. The examinations were directed primarily to the determination of the distribution of the pores in the specimens according to pore sizes and the changes of such distribution. The examinations were repeated after the respective freeze-thaw cycles and made graphical illustrations how the distribution of the pores size and amount have changed as a function of the number of the cycles. The article has established that throughout the cycles, the size of the pores gradually increases to a varying extent, and after the 30^{th} cycle, drastic changes were experienced in the specimens. Based on the data that can be found in the article, one cannot draw any conclusion according to which any correlation would exist between the freeze-thaw resistance and the initial volume of pores, moreover the data supports just the opposite of the existence of such a correlation.

The task of the invention is to provide a non-destructive determination method for the freeze-thaw resistance of the examined concrete specimens, which provides reliable results faster and in a simpler way than what is defined in the mentioned standard.

The attribute "faster" does not only mean the sparing of the high number of freeze-thaw cycles, but also refers to the fact that the examinations can start before the full setting of the concrete that requires 28 days.

For solving this task, we have started from the fact that there were several publications in which medical computer tomography (CT) was used instead of micro CT equipment for the examination of stones, asphalts and even of concrete. The advantage of using medical CTs lies in their wide range of use and availability, furthermore the circumstance that the examinations do not limit the size of the specimens and such specimens can have a diameter greater than 75 mm or can have a rectangular or oblong shape or even a square-shaped cross-sectional profile, whereas the properties of such larger specimens can better represent the structure of the concrete to be examined.

Moreover, medical CTs are appropriate for the examination of bones, which have a Hunsfield coefficient close to that of concrete.

In medical CT equipments, the specimen is stationary, and the X-ray source and the detectors are rotating around the specimen which is just the opposite of the arrangements of micro-CT material examinations in which the specimen is rotated, and the X-ray source and the detectors are stationary. The information provided by the detectors is however disturbed by a number of things, and for the elimination or the decrease of such disturbing effects, several mathematical solutions have become available.

A method for improving the picture quality of CT images has been published in the article of Kristóf Kapitány and Árpád Bari: "Fourier Transformation-Based CT Correction Method for Geomaterial Core Samples" (J. Mater. Civ. Eng., 2016,28(1): 06015005) concerning the elimination or minimizing the problems coming from the hardening of the rays and from other noises as well as concerning the separation of zones with differing X-ray attenuations in the different materials. Here, Fourier transformation was carried out to enter the frequency domain, wherein a special second order Butterworth low pass filter was used to screen the high frequency components, and by means of a fast inverse Fourier transformation, a returning to the real domain was reached. The results thus obtained made the components of the specimens with differing attenuation suitable to be processed separately and to be displayed in a distinguishable way. The authors have elaborated this method for the examination of specimens made from asphalt and concrete, and this method is appropriate to display air voids present in the specimen and to determine the total volume of the air voids, namely the pores.

For the examination of the pores present in asphalt and concrete, not only the previously referred picture enhancing method is known, but there are numerous other solutions which are included in the article by Éva Lublóy et, al entitled: "Air Void Distribution of Asphalts Determined by Computer Tomography" (Period. Polytech. Civil Eng. 2015, pp. 503-510) and also listed among the references of the article. The article describes that the correct nature of the results obtained by the CT examinations were verified by conventional measurements, and the knowledge of the internal structure of asphalt can have a substantial role in checking and improving its quality.

CHEN SHAOJIE ET AL: "Salt Freeze-Thaw Damage Characteristics of Concrete based on Computed Tomography",TEHNICKI VJESNIK, vol. 26, no. 6, 1 November 2019 (2019-11-01), pages 1753-1763, ISSN: 1330-3651, DOI: 10.17559/TV-20190819080524 discloses the investigation of the pore distribution of concrete samples using a medical grade computed tomographic inspection apparatus with subsequent 3D pore volume calculation of the reconstructed images in order to ascertain the damage to the samples after 200 freeze-thaw cycles.

For solving the task, it has been recognized that the freeze-thaw resistance depends basically on the full volume of the air voids (pores) in the concrete, and good results can be obtained if the examinations are carried out under real conditions, i.e. the specimens with scratches or having too many pores are left out of the examinations and the air voids in the aggregates are left out from the calculations. Such examinations can be carried out in the most convenient way by means of medical CT equipments following a high-quality picture correction.

A further essential recognition has been that the pore structure is formed already by the end of the sixth day following the manufacture of the concrete structure (and of the specimen), and even if it changes, such change will be negligibly low and act in the direction of the decrease of the pore volume, therefore the examinations can already be carried out on the 6^{th} or 7^{th} day after manufacture. This represents a substantial gain in time compared to any known freeze-thaw examination methods.

For solving the task, a method has been provided for the examination of the freeze-thaw resistance of concrete structures, comprising the steps as defined in the attached claims.

The invention will now be described in connection with preferred embodiments thereof in which reference will be made to the accompanying drawings. In the drawing:
Fig. 1 shows the functional layout of the arrangement;
Fig. 2 shows the picture of a slice that comprises air voids in the aggregate;
Fig. 3 shows an enlarged detail of Fig. 2;
Fig. 4 shows is a sectional view showing a crack;
Fig. 5 shows a version of the picture of Fig. 4 processed by a picture correction;
Figs. 6 and 7 show the sectional view of a non-acceptable sample and an enlarged detail thereof;
Figs. 8 and 9 show the sectional view of a sample having acceptable freeze resistance according to the XF1 and XF2 conditions and its enlarged detail; and
Figs. 10 and 11 show the sectional view of a sample having acceptable freeze resistance according to the XF3 and XF4 conditions and its enlarged detail.

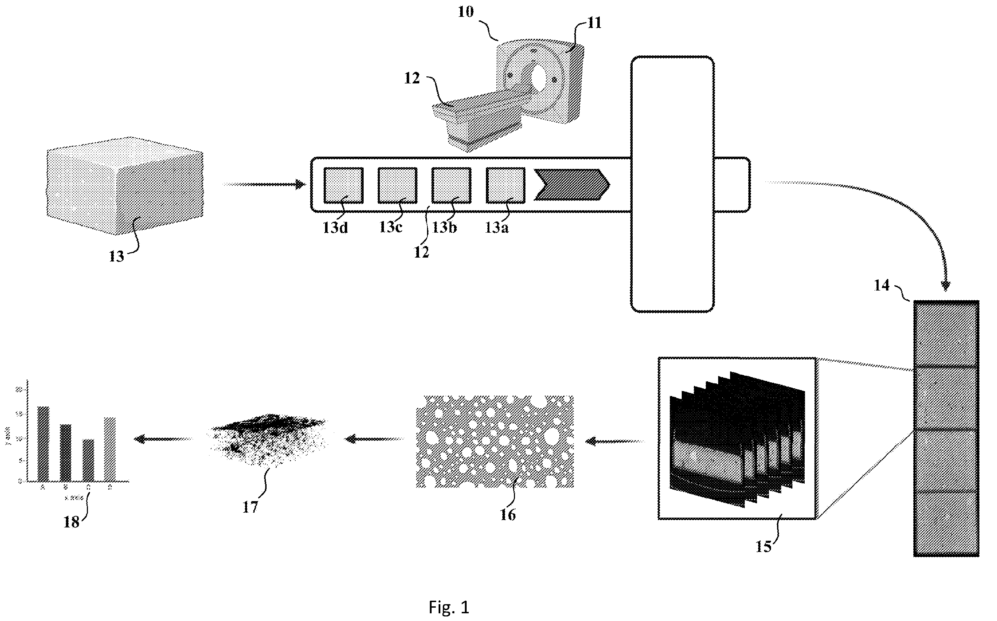

Reference is made now to Fig. 1 in which the functional layout of the arrangement has been shown which is used for the examination of the specimens. A medical computer tomograph 10 (CT) has a stationary part 11 which has a ring-like opening. A movable object table 12 is provided that can be moved in axial direction across the ring like opening, onto which one or more specimens 13 can be placed. In the present case, the specimen 13 has been prepared in the same way as the concrete to be examined and it has an oblong-shaped body having the same properties as the concrete under examination and it has a size preferably 150 x 150 x 1000 mm. It is preferred if the specimen 13 does not comprise more than 5V% component with a density over 3 g/cm³.

When the specimen 13 is placed onto the object table 12 and the medical CT 10 is set to operation, an X-ray generator arranged in the interior of the stationary part 11 and detectors arranged opposite thereto will rotate with high speed, and during each full revolution the detectors sense the radiation passed trough at a given slice of the specimen 13 which has been passed through the specimen 13 and have become attenuated thereby, and a corresponding set of measured data become recorded. During the movement of the specimen, this process is repeated and as a result a segmented set of signals is generated which is recessed by the medical CT 10 and as a result in each slice or segment of the specimen respective sectional pictures are obtained, which are visualized in Fig. 1 by the set of pictures 14 positioned under each other. Fig.

In view of the fact that the pictures 14 correspond to respective subsequent discrete cross sections of the specimen 13, when the pictures are positioned on the top of each other, a virtual reconstruction 15 of the specimen 13 will be available. In case these pictures 14 are appropriately screened, corrected and processed, then we will obtain the reconstructed inner structure 16 of the respective cross sections of the specimen 13, in which the components constituting the specimen 13 will separately appear according to their respective densities and can be separately examined. By means of an appropriate electronic screening, one can provide the spatial model 17 of the respective components like aggregates, steel-reinforcements, cement and air voids present in the specimen 13 which is illustrated in Fig 1 by a symbolic brick shape, but by means of a computer a diagram 18 can also be prepared showing the respective components with differing densities, wherein the respective columns can show the relative volume of the concerned components.

The processing and analysis of the respective cross-sectional pictures can be carried out by algorithms made in a digital environment, which use previously set parameters to segment the aggregates, binders, gaps and pores by thresholding. The mentioned digital environment can be preferably Matlab, but it can be substituted by any other appropriate mathematical algorithm program. Such a digital thresholding algorithm can be the previously mentioned double Fourier transformation.

From the improved quality cross sectional pictures, percentual volume statistics are provided from slice to slice. The respective volume statistics of the slices can be united in a statistic which is characteristic to the specimen that contains sufficient amount and quality data concerning the internal structure of the examined concrete specimen.

The determination of the full porosity value can take place in a separate way or within a general algorithm which carries out the tasks of thresholding one by one, the calculation of the respective percentual volumes, and their summing up.

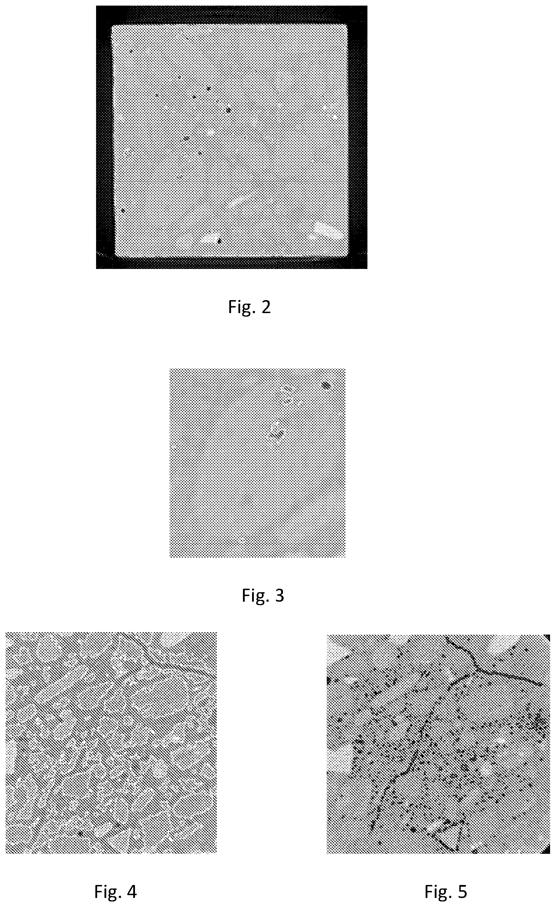

For the examination of the freeze-thaw resistance only selected specimens are appropriate. It is known that concrete comprises a predetermined amount of aggregates that comprise at least in part gravel or crushed stone. These aggregates can also contain air voids which are detected by the examination, whereas such air voids are entirely encircled by the stone material and water cannot penetrate into them, and in this way the mechanical expansion during freezing cannot have any destroying effect. Figs. 2 and 3 show a reconstructed picture of a cross section of the specimen in which the grey shade of the aggregate is darker, and any air void or pore therein has a differing shade. Fig. 3 is the enlarged picture of a detail that comprises such an air void from which one can see that the air void is surrounded in every direction by the material of the stone, whereby water that might penetrate into the interior of the specimen cannot reach such air voids.

When determining the total pore volume in the specimen 13, the summed volume of such air voids in the aggregates must be deducted. Naturally, if in any given specimen the aggregates do not comprise such air voids or their total volume is negligibly low, then this deduction step can be omitted.

A further condition for the examination of freeze-thaw resistance only such specimens 13 can be used in which the total pore volume is under a threshold level, because in case of largely porous concretes one cannot interpret freeze-thaw resistance at all, since water can easily penetrate into the pores, and following the subsequent freeze-thaw cycles, the concrete gets destroyed. Such concretes should be excluded for the examinations. A condition of such an exclusion lies in that the total pore volume is above 20% compared to the full volume.

A third condition of excluding a specimen from the examinations is that it cannot comprise cracks in which the penetrating water can cause serious damages when being frozen. Fig. 4 is the cross-sectional picture of a specimen that includes cracks in which the crack is not as visible as Fig. 5 made from the same slice wherein during picture processing parts containing air are shown as black spots. This cross-sectional view clearly indicates the existence of cracks. The longitudinal size of the cracks can be obtained by the analysis of subsequent adjacent slices and the spatial interpolation of the void parts, which can be done by appropriate available software.

A further condition of excluding a specimen from the examination lies in when it comprises more components (i.e. steel) with a density above 3 g/cm³ which are above an upper limit being e.g. 5 V%.

Before the freeze-thaw resistance examinations of a specimen 13, the data obtained by the medical CT examinations should be screened by mathematical methods e.g. by fast Fourier transformation, then by a Butterworth filtering the by inverse Fourier transformation, then it should be decided from slice to slice whether the specimen comprises cracks that exclude the use thereof, and if such are not found, the volume of the air voids present in the aggregates should be determined and deducted from the full pore volume, and it should be established whether it is in the accepted range. If this is under the threshold, then based on the so determined total pore volume, the freeze-thaw resistance can be established.

The basis of the present invention was the assumption that if from the specimens of the concrete to be examined the non-useable specimens are excluded, and the pore volume is corrected by the deduction of the volume of the air voids in the aggregates, then, based on the percentual volume of the pores, the freeze-thaw resistance can be established.

For confirming this assumption, a high number of examinations were carried out, wherein from the same concrete material the percentual pore volumes were measured by the referred examinations by means of medical CT devices, and separate examinations were carried out following the European standard CRN/TS 12390-9 by capillary suction, and the results obtained were compared with each other. Based on this comparison, a high degree of correlation was obtained on the basis of which it can be established that the freeze-thaw resistance can be determined on the basis of the full pore volume of the specimen.

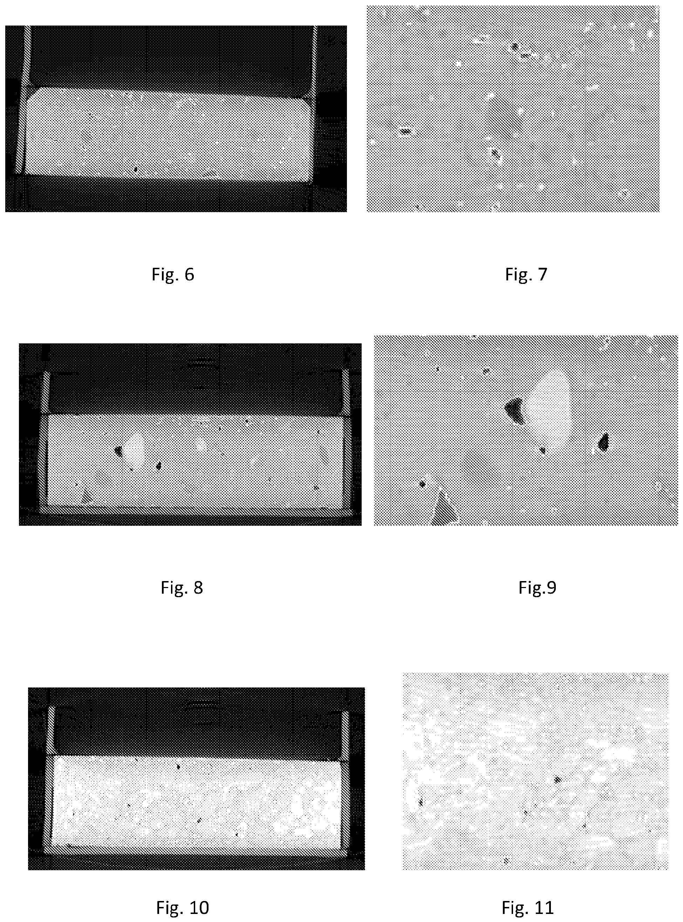

The results obtained are reported based on Figs. 6 to 11. In case the pore volume in the specimen is above 6 V%, there can be no freeze-thaw resistance and the concrete does not satisfy the conditions set in the standard. Fig. 6 shows a cross sectional picture of a sample with 9.5V% pore volume, and Fig. 7 is an enlarged detail of the same picture. The specimen comprises comparatively many pores into which water can penetrate and when getting frozen, its volume expansion at freezing will increase the available pore volume, and during the subsequent freeze-thaw cycles a gradual worsening takes place and thus the mechanical strength and loadability of the concrete worsens. In the pore volume range 6 V%- 20 V% the specimen does not have freeze-thaw resistance.

Figs. 8 and 9 show the cross-sectional pictures of a specimen with 5 V% pore volume and an enlarged detail thereof. In case the pore volume determined by the CT examination is between 4 V% and 6 V%, then the concrete is moderately freeze-thaw resistant and the standard qualification XF1 and XF2 can be applied.

Freeze-thaw resistance is present in case of concretes with pore volume under 4 V%. Figs. 10 and 11 show the cross-sectional pictures of a specimen with 2.5 V% pore volume and an enlarged detail thereof. It should be noted that, as explained earlier, the air voids that can be observed in the aggregate are not taken into account when the pore volume is determined.

Similar pore volume examinations were made by the previously described CT technique after the specimens were kept under water through a few days under a pressure of 5 bars. Because the Hunsfield coefficient of water substantially differs from the coefficients of concrete and of the aggregates, pores filled with water can be separated in the corrected sectional pictures the same way as in case of air, as the measured pore volume did not differ in a remarkable way from the results obtained in dry conditions.

It has also been examined whether the pore volume of a specimen changes if the examination is carried out on the 7^{th} day following manufacture and not on or after the 28^{th} day. It has been experienced that the results do not change with time, or the pore volume determined in the 28^{th} day is slightly lower (by less than 1V%) than the values obtained on the 7^{th} day. Therefore if a specimen proves to be freeze-thaw resistance by an examination taken on the 7^{th} day, it will only be better on the 28^{th} day, therefore the examinations can be carried out from the 7^{th} day onwards.

The examination method can be complemented by a further step in which from the sectional views a 3D model is created by means of known computer-based picture and model creating programs. The so obtained structural model can provide a basis for carrying out further diagnostic examinations including the examination of the spatial distance-coefficient obtained from the distance between the pores, and other important examinations.

The method according to the invention has really solved the task of the invention because the freeze-thaw resistance can be obtained in a reliable way already on the 6^{th} or 7^{th} day. In addition to the quick nature, further advantages come from the simplicity and smaller costs of the suggested method. The use of a medical CT device is a substantial advantage, as such devices are available in many places and they allow the examination of larger specimens.

As a result of the examinations, the spatial structure of the specimens will be available in a form that can be further examined by a computer and displayed as a visual image that can be important concerning further structural examinations. Such a further processing is facilitated by the fact that the utilized picture correction methods enable accurate and well detectable processing and displaying the respective components.

## Claims

1. Method for the examination of the freeze-thaw resistance of concrete structures, comprising the steps of preparing one or more specimens (13) of a predetermined size from the concrete structure when it is made that represents the concrete structure, examining the inner structure of the specimen by computer tomography (10) and determining the relative amount of a predetermined component in the specimen following picture enhancement and picture correction carried out by a computer, **characterized in that** said examination is carried out after six days have elapsed following the preparation of the specimen (13), then checking whether the specimen is appropriate for the examination of freeze-thaw resistance qualifying as inappropriate for examination in case the total pore volume therein is above 20 V%, or where it comprises cracks, or if the amount of the components therein that have a density over 3 g/cm³ exceeds 10 V%, and in case if it is found that the specimen (13) is suitable for such an examination, determining the percentual volume of the pores in the portion of the specimen not counting the pores in the aggregate components, and in case the percentual volume of the pores is above 6 V%, the specimen is qualified as having no freeze-thaw resistance, if this value is between 6 V% and 4 V%, the specimen is qualified as moderately freeze-thaw resistant, and if this value is under 4 V%, the specimen is qualified as freeze-thaw resistant.

2. The method as claimed in claim 1, wherein when the calculation of the amount of pores the volume of pores in the aggregates is determined and this value is deducted from the measured total pore volume.

3. The method as claimed in claims 1 or 2, wherein the examination is carried out in the period between 7^{th} to 28^{th} days following the preparation of the specimen (13).

4. The method as claimed in any of the claims 1 to 3, wherein the specimen is a right prism with square base, wherein the size of the square is between 80 and 200 mm and the length of the prism is under 1000 mm.

5. The method as claimed in any of the claims 1 to 4, wherein during said picture enhancement and picture corrections steps the signal series obtained from the detectors of the medical computer tomograph (10) are subjected to a Fourier transformation then passed through a low pass filter and finally an inverse Fourier transformation is carried out.

6. The method as claimed in any of the claims 1 to 5, wherein the examinations are carried out on the 7^{th} day after the preparation of the specimen (13).

## Patentansprüche

1. Verfahren zur Untersuchung der Frost-Tau-Beständigkeit von Betonstrukturen, umfassend die Schritte des Herstellens einer oder mehrerer Proben (13) einer vorbestimmten Größe aus der Betonstruktur, wenn diese hergestellt wird, die die Betonstruktur darstellt, des Untersuchens der inneren Struktur der Probe durch Computertomographie (10) und des Bestimmens der relativen Menge einer vorbestimmten Komponente in der Probe nach Bildverbesserung und Bildkorrektur, die durch einen Computer durchgeführt werden, **dadurch gekennzeichnet, dass** die Untersuchung nach Ablauf von sechs Tagen nach der Herstellung der Probe (13) durchgeführt wird, dann überprüft wird, ob die Probe für die Untersuchung der Frost-Tau-Beständigkeit geeignet ist, wobei sie als ungeeignet für die Untersuchung gilt, falls das Gesamtporenvolumen darin über 20 V% liegt, oder wenn sie Risse enthält, oder wenn die Menge der Komponenten darin, die eine Dichte über 3 g/cm³ aufweisen, 10 V% übersteigt, und falls festgestellt wird, dass die Probe (13) für eine solche Untersuchung geeignet ist, das prozentuale Volumen der Poren in dem Teil der Probe bestimmt wird, wobei die Poren in den Aggregatkomponenten nicht mitgezählt werden, und falls das prozentuale Volumen der Poren über 6 V% liegt, wird die Probe als nicht Frost-Tau-beständig eingestuft, falls dieser Wert zwischen 6 V% und 4 V% liegt, wird die Probe als mäßig Frost-Tau-beständig eingestuft, und falls dieser Wert unter 4 V% liegt, wird die Probe als Frost-Tau-beständig eingestuft.

2. Verfahren nach Anspruch 1, wobei bei der Berechnung der Porenmenge das Porenvolumen in den Aggregaten bestimmt und dieser Wert vom gemessenen Gesamtporenvolumen abgezogen wird.

3. Verfahren nach Anspruch 1 oder 2, wobei die Untersuchung im Zeitraum zwischen dem 7. und 28. Tag nach der Herstellung der Probe (13) durchgeführt wird.

4. Verfahren nach einem der Ansprüche 1 bis 3, wobei die Probe ein gerades Prisma mit quadratischer Grundfläche ist, wobei die Größe des Quadrats zwischen 80 und 200 mm und die Länge des Prismas unter 1000 mm liegt.

5. Verfahren nach einem der Ansprüche 1 bis 4, wobei während der Bildverbesserungs- und Bildkorrekturschritte die von den Detektoren des medizinischen Computertomographen (10) erhaltenen Signalreihen einer Fouriertransformation unterzogen werden, anschließend durch einen Tiefpassfilter geleitet werden und abschließend eine inverse Fouriertransformation durchgeführt wird.

6. Verfahren nach einem der Ansprüche 1 bis 5, wobei die Untersuchungen am 7. Tag nach der Herstellung der Probe (13) durchgeführt werden.

## Revendications

1. Procédé d'examen de la résistance au gel-dégel des structures en béton, comprenant les étapes de préparation d'un ou plusieurs spécimens (13) d'une taille prédéterminée à partir de la structure en béton lorsqu'elle est fabriquée qui représente la structure en béton, l'examen de la structure interne du spécimen par tomographie par ordinateur (10) et la détermination de la quantité relative d'un composant prédéterminé dans le spécimen à la suite de l'amélioration de l'image et de la correction de l'image effectuées par un ordinateur, **caractérisé en ce que** ledit examen est effectué après que six jours se sont écoulés après la préparation du spécimen (13), puis en vérifiant si le spécimen est approprié pour l'examen de la résistance au gel-dégel, qualifié d'inapproprié pour l'examen dans le cas où le volume total des pores dans l'échantillon est supérieur à 20 V %, ou s'il comprend des fissures, ou si la quantité des composants qui ont une densité supérieure à 3 g/cm³ dépasse 10 V %, et s'il est constaté que le spécimen (13) est approprié pour un tel examen, la détermination du volume en pourcentage des pores dans la partie du spécimen ne comptant pas les pores dans les composants agrégés, et si le volume en pourcentage des pores est supérieur à 6 V %, le spécimen est qualifié comme n'ayant pas de résistance au gel-dégel, si cette valeur est comprise entre 6 V % et 4 V %, le spécimen est qualifié comme modérément résistant au gel-dégel, et si cette valeur est inférieure à 4 V %, le spécimen est qualifié comme résistant au gel-dégel.

2. Procédé selon la revendication 1, dans lequel, lors du calcul de la quantité de pores, le volume de pores dans les agrégats est déterminé et cette valeur est déduite du volume total des pores mesuré.

3. Procédé selon la revendication 1 ou 2, dans lequel l'examen est effectué dans la période comprise entre le 7^{ème} et le 28^{ème} jour après la préparation du spécimen (13).

4. Procédé selon l'une quelconque des revendications 1 à 3, dans lequel le spécimen est un prisme droit à base carrée, dans lequel la taille du carré est comprise entre 80 et 200 mm et la longueur du prisme est inférieure à 1000 mm.

5. Procédé selon l'une quelconque des revendications 1 à 4, dans lequel, au cours desdites étapes d'amélioration et de correction de l'image, les séries de signaux obtenues à partir des détecteurs du tomographe médical par ordinateur (10) sont soumises à une transformation de Fourier, puis passent à travers un filtre passe-bas et, enfin, une transformation de Fourier inverse est effectuée.

6. Procédé selon l'une quelconque des revendications 1 à 5, dans lequel les examens sont effectués le 7^{ème} jour après la préparation du spécimen (13).
